# EUROPEAN PATENT SPECIFICATION

(11) **EP 4 494 223 B1**
(45) Date of publication and mention of the grant of the patent: **17.12.2025**
(21) Application number: 23720358.3
(22) Date of filing: 14.03.2023
(51) Int. Cl.: H01S 5/042, H01S 5/183, H01S 5/32, H01S 5/343

(54) **SURFACE EMITTING LASER, PROJECTION APPARATUS, HEAD-UP DISPLAY, MOVING BODY, HEAD-MOUNTED DISPLAY, AND OPTOMETRY APPARATUS**
OBERFLÄCHENEMITTIERENDER LASER, PROJEKTIONSVORRICHTUNG, HEAD-UP-ANZEIGE, BEWEGLICHER KÖRPER, KOPFMONTIERTE ANZEIGE UND OPTOMETRIEVORRICHTUNG
LASER À ÉMISSION PAR LA SURFACE, APPAREIL DE PROJECTION, AFFICHAGE TÊTE HAUTE, CORPS MOBILE, VISIOCASQUE ET APPAREIL D'OPTOMÉTRIE

(30) Priority: 18.03.2022 JP 2022044205; 30.11.2022 JP 2022192216
(43) Date of publication of application: 22.01.2025
(73) Proprietor: Ricoh Company, Ltd., Tokyo 143-8555 (JP)
(72) Inventor: KAMINISHI, Morimasa, Tokyo 143-8555 (JP); IWATA, Hirokazu, Tokyo 143-8555 (JP)
(74) Representative: J A Kemp LLP
(86) International application number: PCT/IB2023/052426
(87) International publication number: WO 2023/175482

(56) References cited:
- WO-A1-2009/047901
- US-A1- 2005 249 254
- US-A1- 2006 060 876
- US-A1- 2009 041 075
- US-A1- 2021 028 603
- US-B1- 8 774 246
- US-B2- 10 116 115
- US-B2- 6 697 405

## Description

### [Technical Field]

Embodiments of the present disclosure relate to a surface emitting laser, a projection apparatus, a head-up display (HUD), a mobile object, a head-mounted display (HMD), and an optometry apparatus.

### [Background Art]

A vertical cavity surface emitting laser (VCSEL) is a laser in which a thin active layer is sandwiched between a pair of reflecting mirrors to form a resonator in a direction perpendicular to a substrate. The reflecting mirror may be required to have a reflectance of 99% or more.

In a GaN-based material, it is difficult to form a semiconductor reflector with low resistance as compared with a GaAs-based material. For this reason, when a surface-emitting laser is manufactured using a GaN-based material, an intra-cavity contact structure is often used as a structure for injecting current into an active layer, in which a reflector is not energized and contact is made in a resonator. At this time, a p-side contact is often formed by forming a limited opening (aperture) as a current confinement structure on highly doped p-GaN using a high resistance layer such as a dielectric, and injecting a current by a conductive transparent film such as indium tin oxide (ITO).

In a VCSEL having a current injection structure using ITO, light absorption of ITO might cause issues. Typically, ITO has a light absorption of several thousand cm-1 from the 400 nm band developed in GaN-based VCSELs to the visible light region. For this reason, the influence of absorption is reduced by thinly providing ITO at the node portion of the electric field intensity in the resonator. The ITO thickness is most often less than or equal to 50 nm and often less than or equal to 20 nm.

The resistivity of ITO is usually on the order of 1 × 10-4 Ω cm, and ITO in the lateral direction inevitably has a high resistance. Further, since the resistivity of p-GaN on the injection side is on the order of one digit Ω cm, the current injected from the ITO does not spread substantially in the lateral direction but flows in the resonator in the longitudinal direction toward the active layer. At this time, due to the influence of the thin ITO, it is difficult to make the current density uniform in the peripheral portion and the central portion of the aperture, and as a result, the current injection density into the active layer tends to be high in the peripheral portion and low in the central portion. In particular, the larger the diameter of the aperture, the greater the influence thereof. This makes it difficult for the active layer inside the aperture to obtain a uniform gain, and in particular, makes it difficult to stably obtain a single mode.

Further, Non-Patent Literature (NPL) 1 describes an attempt to equalize current injection into the aperture by making an n-side distributed Bragg reflector (DBR) conductive. However, even in the configuration described in NPL 1, the nonuniformity of the current injection density caused by the high electric resistance of ITO is not solved. US 6 697 405 and WO 2009/047901 disclose background art.

### [Citation List]

### [Non-Patent Literature]

[NPL 1]
Japanese Journal of Applied Physics 59, SGGE08 (2020)

### [Summary of Invention]

### [Technical Problem]

It is an objective of the present disclosure to provide a surface emitting laser, a projection apparatus, a HUD, a mobile object, a HMD, and an optometry apparatus, which achieves higher uniformity of the density of a current injected into a resonator.

### [Solution to Problem]

A surface emitting laser includes: a first reflector; a second reflector; a resonator between the first reflector and the second reflector, the resonator including an active layer; and a conductive layer through which a current is injected into the active layer of the resonator. The resonator further includes: a first layer including a first p-type semiconductor layer having a first band gap, the first layer having a first face contacting the conductive layer and a second face opposite to the first face; and a second layer including a second p-type semiconductor layer having a second band gap larger than the first band gap, the second layer between the first layer and the active layer. The second p-type semiconductor layer contacts the second face of the first layer. The conductive layer contacts at least a part of the first face of the first layer and an interface between the first layer and the second layer.

### [Advantageous Effects of Invention]

Embodiments of the present disclosure achieve higher uniformity of the density of the current injected into the resonator.

### [Brief Description of Drawings]

The accompanying drawings are intended to depict example embodiments of the present invention and should not be interpreted to limit the scope thereof. The accompanying drawings are not to be considered as drawn to scale unless explicitly noted. Also, identical or similar reference numerals designate identical or similar components throughout the several views.
[FIG. 1]

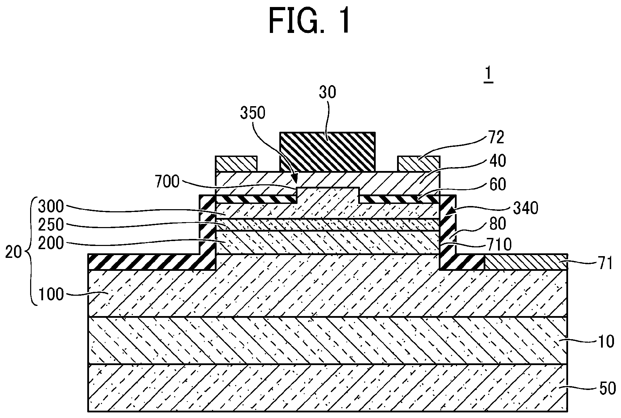

   FIG. 1 is a cross-sectional view illustrating a surface emitting laser according to a first embodiment.
[FIGS. 2A and 2B]

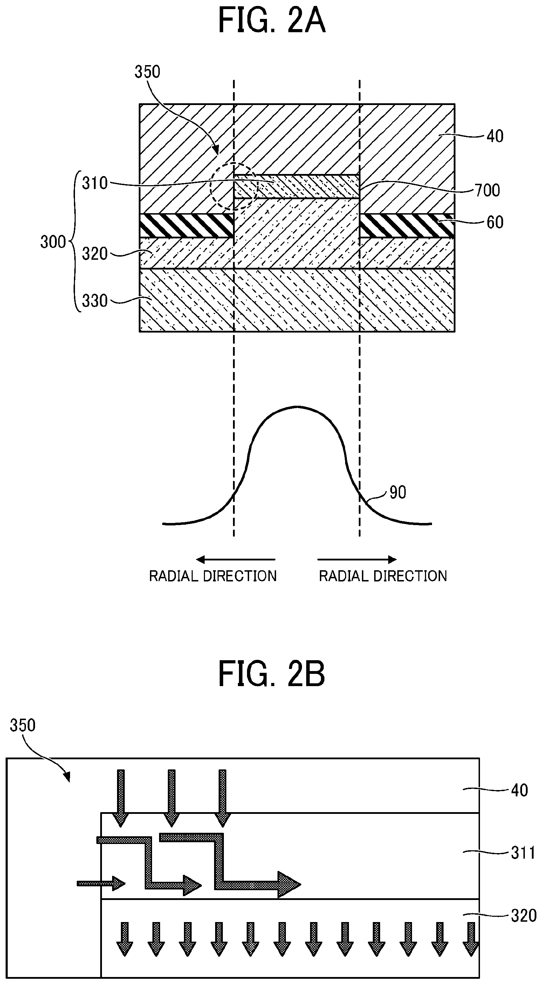

   FIGS. 2A and 2B are cross-sectional views of an area in and around a ridge structure of the surface emitting laser in FIG. 1.
[FIGS. 3A and 3B]

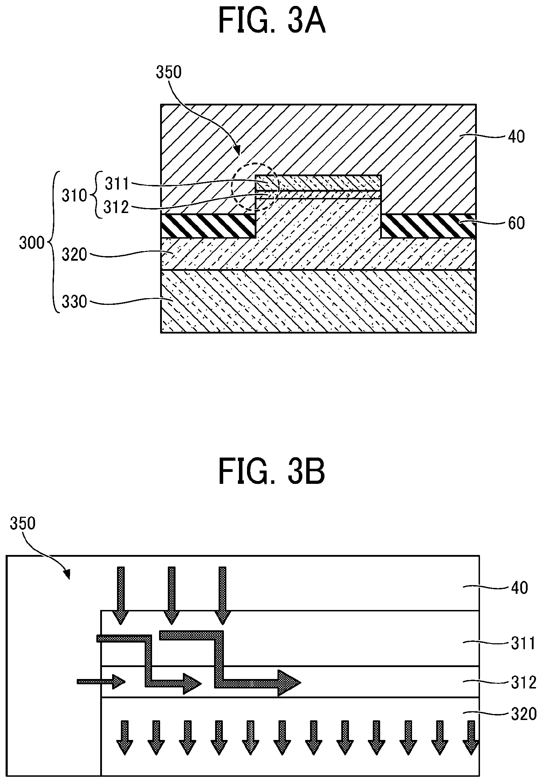

   FIGS. 3A and 3B are cross-sectional views of an area in and around a ridge structure of a surface emitting laser according to a second embodiment of the present disclosure.
[FIG. 4]

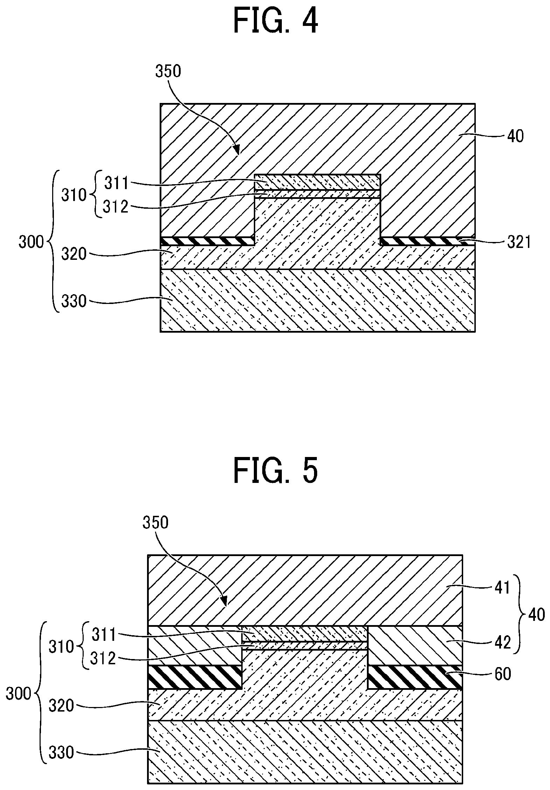

   FIG. 4 is a cross-sectional view of an area in and around a ridge structure of a surface emitting laser according to a third embodiment of the present disclosure.
[FIG. 5]
   FIG. 5 is a cross-sectional view of an area in and around a ridge structure of a surface emitting laser according to a fourth embodiment of the present disclosure.
[FIGS. 6A and 6B]

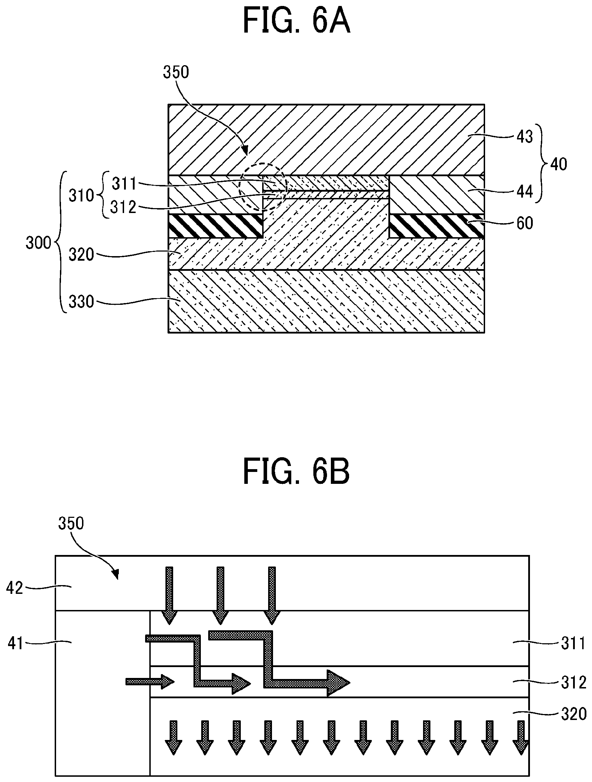

   FIGS. 6A and 6B are cross-sectional views of an area in and around a ridge structure of a surface emitting laser according to a fifth embodiment of the present disclosure.
[FIG. 7]

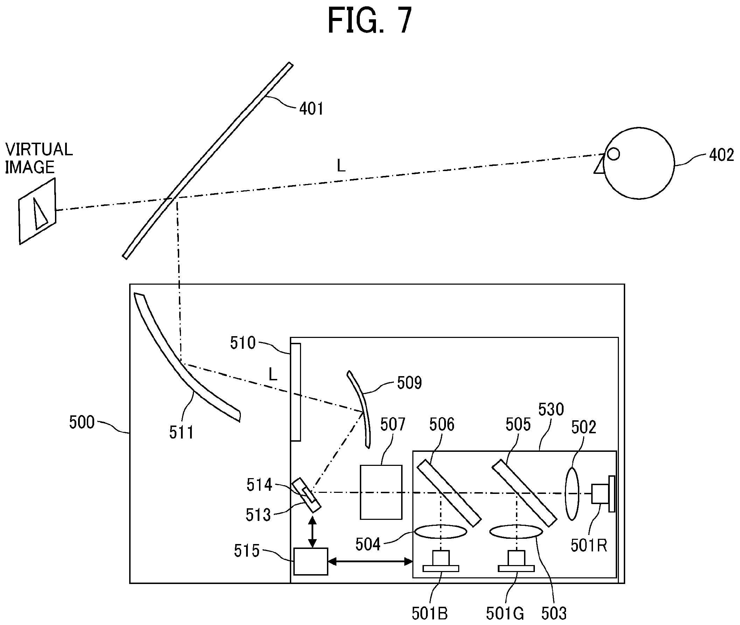

   FIG. 7 is a diagram of a HUD as an example of a projection apparatus according to a sixth embodiment.
[FIG. 8]

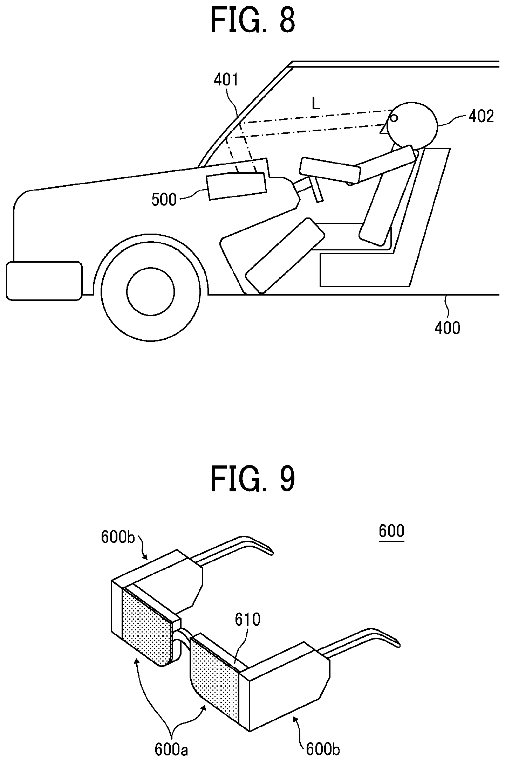

   FIG. 8 is a diagram of a vehicle mounted with the HUD in FIG. 7.
[FIG. 9]
   FIG. 9 is an external view of a HMD according to the seventh embodiment.
[FIG. 10]

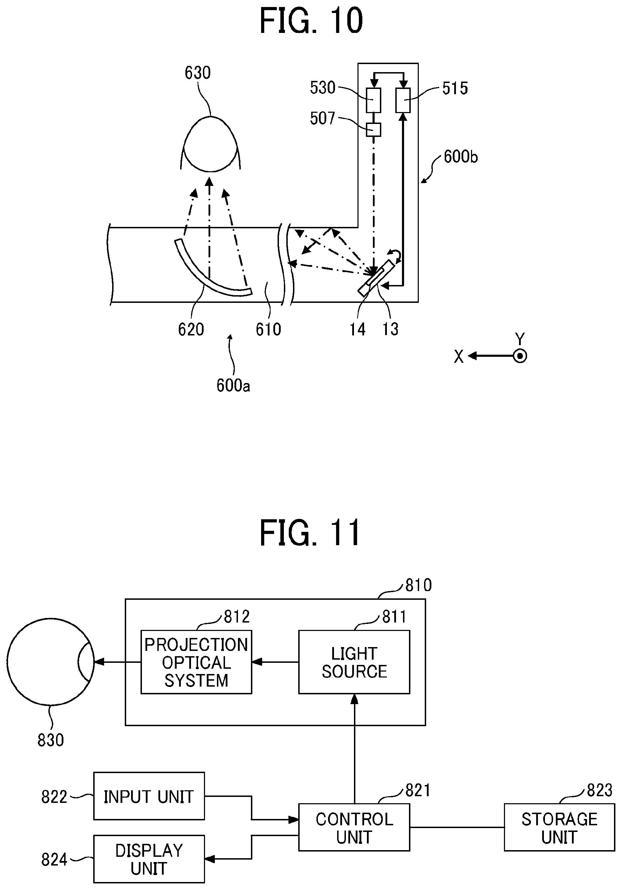

   FIG. 10 is a diagram of a part of a configuration of a HMD in FIG. 9.
[FIG. 11]
   FIG. 11 is a block diagram of an optometry apparatus according to an eighth embodiment.

### [Description of Embodiments]

The terminology used herein is for the purpose of describing particular embodiments only and is not intended to be limiting of the present invention. As used herein, the singular forms "a," "an," and "the" are intended to include the plural forms as well, unless the context clearly indicates otherwise.

In describing embodiments illustrated in the drawings, specific terminology is employed for the sake of clarity. However, the disclosure of this specification is not intended to be limited to the specific terminology so selected.

Embodiments of the present disclosure are described below with reference to the accompanying drawings. In the specification and the drawings, components having substantially the same functional configuration are denoted by the same reference sign, and redundant description may be omitted.

### First Embodiment

First, the first embodiment will be described. The first embodiment relates to a surface emitting laser. FIG. 1 is a cross-sectional view illustrating a surface emitting laser 1 according to the first embodiment. FIGS. 2A and 2B are cross-sectional views of an area in and around a ridge structure of the surface emitting laser 1 according to the first embodiment. FIG. 2A is a cross-sectional view or an enlarged view of a part of the surface emitting laser 1 in FIG. 1. FIG. 2B is a cross-sectional view of a portion surrounded by a broken-line circle in FIG. 2A, illustrating currents indicated by arrows in and around the portion.

As illustrated in FIG. 1, the surface emitting laser 1 according to the first embodiment includes a substrate 50, a lower reflector 10 (a first reflector), a resonator 20, a conductive layer 40, and an upper reflector 30 (a second reflector). The lower reflector 10 is on the substrate 50, resonator 20 is on the lower reflector 10, the conductive layer 40 is on the resonator 20, and the upper reflector 30 is on the conductive layer 40. The surface emitting laser 1 is configured to output light having a wavelength of 450 nm.

The substrate 50 is, for example, a GaN substrate having a c-plane upper surface. The lower reflector 10 is, for example, a DBR made of a semiconductor material.

The resonator 20 includes a lower spacer layer 100, an active layer 200, and an upper spacer layer 300. The lower spacer layer 100 is on the lower reflector 10, the active layer 200 is on the lower spacer layer 100, and the upper spacer layer 300 is over the active layer 200. An electron-blocking layer 250 having a thickness of approximately 20 nm between the active layer 200 and the upper spacer layer 300. Within the context of the present disclosure, if a first layer is stated to be "over" a second layer, the first layer may be in direct contact with a portion or all of the second layer, or there may be one or more intervening layers between the first and second layer. The resonator 20 is an example of a semiconductor laminated structure.

For example, the lower spacer layer 100 is an n-type GaN layer doped with, for example, Si at a concentration of 3 × 10¹⁸/cm³. The active layer 200 has a multiple quantum well structure including multiple InGaN layers and multiple GaN layers. The active layer 200 emits light having a wavelength of 450 nm. The electron-blocking layer 250 is, for example, an A_{l0.2}Ga_{0.8}N layer doped with Mg at a concentration of 2 × 10¹⁹/cm³, for example.

As illustrated in FIG. 2A, the upper spacer layer 300 includes a first layer 310, a second layer 320, and a third layer 330. The second layer 320 is on the third layer 330, and the first layer 310 is on the second layer 320. The third layer 330 is closest to the active layer 200 among the layers of the upper spacer layer 300. The third layer 330 is a p-type GaN layer doped with, for example, Mg at a concentration of 2 × 10¹⁹/cm³. The second layer 320 is a p-type Al_{0.05}Ga_{0.95}N layer doped with, for example, Mg at a concentration of 2 × 10¹⁹/cm³. The second layer 320 has a thickness of 10 nm, for example. The first layer 310 is a p-type In_{0.07}Ga_{0.93}N layer doped with, for example, Mg at a concentration of 2 × 10¹⁹/cm³. The first layer 310 has a thickness of 15 nm, for example. However, an outermost layer portion at a depth of 10 nm from the upper surface of the first layer 310 is doped with Mg at a higher concentration, for example, at a concentration of 3 × 10²⁰/cm³. The band gap (a second band gap) of the second layer 320 is larger than the band gap (a first band gap) of the first layer 310. In the present embodiment, the second layer 320 is an example of a second p-type semiconductor layer, and the first layer 310 is an example of a first p-type semiconductor layer.

A mesa structure 340 is formed in the upper spacer layer 300, the active layer 200, and the lower spacer layer 100. The mesa structure 340 is formed at such a height that the lower spacer layer 100 is exposed around the mesa structure 340. The mesa structure 340 is formed in a cylindrical shape having a diameter of 25 µm.

A ridge structure 350 is formed in the upper spacer layer 300. The ridge structure 350 is formed at such a height that the second layer 320 is exposed around the ridge structure 350. For example, the height of the ridge structure 350 is 20 nm. The ridge structure 350 is formed in a columnar shape, for example, a shape of a circular column or a polygonal prism having a diameter of 10 µm. A dielectric layer 60 is formed on the second layer 320 exposed from the ridge structure 350. The dielectric layer 60 is in contact with a side surface of a portion of the second layer 320 that forms the ridge structure 350. The upper surface of the dielectric layer 60 is located closer to the active layer 200 than the interface between the first layer 310 and the second layer 320. The dielectric layer 60 is, for example, a SiO₂ layer. A laminated body, in which the first layer 310 and a portion of the second layer 320 is laminated in a lamination direction, has the ridge structure 350 having a columnar shape. The first layer 310 has a first outer periphery 700. The active layer 200 has a second outer periphery 710. The first outer periphery 700 of the first layer 310 is interior of the second outer periphery 710 of the active layer 200 in a radial direction of the ridge structure 350 as illustrated in FIGS. 1 and 2.

The conductive layer 40 is provided on the dielectric layer 60 and the ridge structure 350. The conductive layer 40 is in contact with the entire upper surface of the first layer 310 and the interface between the first layer 310 and the second layer 320. The conductive layer 40 is a transparent conductive layer such as an indium-tin oxide (ITO) layer, for example.

The upper reflector 30 is disposed so as to overlap the ridge structure 350 in a plan view of the upper reflector 30. The upper reflector 30 is, for example, a dielectric DBR.

On the n-type lower spacer layer 100 exposed from the mesa structure 340, an n-side electrode 71 in ohmic contact with the lower spacer layer 100 is formed. The n-side electrode 71 has, for example, a laminated film in which a Ti film, an Al film, a Pt film, and an Au film are laminated in this order from the bottom. A dielectric layer 80 is formed on a portion of the n-type lower spacer layer 100 other than a portion where the n-side electrode 71 is formed and on the side surface of the mesa structure 340. The dielectric layer 80 is, for example, a SiN layer.

Around the upper reflector 30, a p-side electrode 72 is formed on the conductive layer 40. The p-side electrode 72 has, for example, a laminated film in which a Ti film and an Au film are laminated in this order from its bottom.

Note that it is preferable that the node of the electric field strength is located in the vicinity of the portion of the first layer 310 doped at a high concentration or in the vicinity of the conductive layer 40.

A method for manufacturing the surface emitting laser 1 according to the first embodiment is described below.

The lower reflector 10, the lower spacer layer 100, the active layer 200, and the upper spacer layer 300 are formed on the substrate 50 by metal organic chemical vapor deposition (MOCVD).

The mesa structure 340 is formed in the laminated layers of the upper spacer layer 300, the active layer, and the lower spacer layer 100. In the formation of the mesa structure 340, an etching mask is formed on the upper spacer layer 300 by photolithography using photoresist or metal, and the laminated layers of the upper spacer layer 300, the active layer, and the lower spacer layer 100 are dry-etched. At this time, the dry etching is stopped halfway in the lower spacer layer 100 in the thickness direction of the lower spacer layer 100.

In the vicinity of the mesa structure 340, the n-side electrode 71 is formed on the lower spacer layer 100 exposed from the mesa structure 340. The n-side electrode 71 can be formed using lift-off. After forming the metallic film constituting the n-side electrode 71, annealing is performed at 550°C in an N₂ atmosphere so that the n-side electrode 71 is in ohmic contact with the lower spacer layer 100.

Next, a dielectric layer 80 is formed over the lower spacer layer 100 to cover the mesa structure 340 and the n-side electrode 71. Next, a resist mask that causes only the upper surface of the mesa structure 340 to be exposed is formed by, for example, photolithography, which is used to remove the dielectric layer 80 by wet etching. Then, a ridge structure 350 is formed in the upper spacer layer 300. In the formation of the ridge structure 350, a resist mask is formed on the upper spacer layer 300 by photolithography, and the upper spacer layer 300 is dry-etched.

Next, the dielectric layer 60 is formed on the upper spacer layer 300 exposed from the ridge structure 350. The dielectric layer 60 has a thickness such that the upper surface of the dielectric layer 60 is closer to the active layer 200 than the interface between the first layer 310 and the second layer 320. Next, a conductive layer 40 is formed over the dielectric layer 60 and the ridge structure 350. In the formation of the dielectric layer 60 and the conductive layer 40, first, the SiO₂ layer and the ITO layer are formed by, for example, sputtering while leaving the resist mask used for forming the ridge structure 350, and then the resist mask is removed. In other words, lift-off is performed. At this time, the thickness of the ITO layer is set such that the upper surface of the ITO layer is substantially flush with the upper surface of the ridge structure 350. After the lift-off, another ITO layer is further formed in contact with the top surface of the ridge structure 350. Thereafter, the excess ITO layer formed on the dielectric layer 80 is removed by, for example, wet etching.

Next, the upper reflector 30 is formed on the conductive layer 40. The upper reflector 30 can be formed by, for example, lift-off. Next, the p-side electrode 72 is formed on the conductive layer 40 around the upper reflector 30. The p-side electrode 72 can be formed by, for example, lift-off. Next, a portion of the dielectric layer 80 on the n-side electrode 71 is removed by dry etching or the like.

In this way, it is possible to manufacture the surface emitting laser 1 according to the first embodiment.

In the surface emitting laser 1 according to the first embodiment, the second layer 320 (for example, an Al_{0.05}Ga_{0.95}N layer) is below the first layer 310 (for example, an In_{0.07}Ga_{0.93}N layer), and the band gap of the second layer 320 is larger than the band gap of the first layer 310. This forms a two-dimensional hole gas (2DHG) in the vicinity of the interface between the first layer 310 and the second layer 320. The 2DHG includes high-concentration carriers, which are two-dimensionally formed in a narrow region having a thickness of approximately 1 nm. The conductive layer 40 is in contact with the entire upper surface of the first layer 310 and the interface between the first layer 310 and the second layer 320.

As illustrated in FIG. 2B, current is injected from the conductive layer 40 into the resonator 20 not only from the upper surface of the first layer 310 but also from the interface between the first layer 310 and the second layer 320. In other words, current is injected from the conductive layer 40 to the resonator 20 through the upper surface and the side wall surface of the ridge structure 350. In addition, since the electric resistance of the 2DHG is lower than the electric resistances of the first layer 310 and the second layer 320, carriers easily spread in the lateral direction. In this configuration, the current injected from the upper surface of the ridge structure 350 spreads in the lateral direction at the 2DHG portion, and the current injection density tends to become uniform inside the aperture. In other words, the variation in the current injection density inside the ridge structure 350 is reduced.

As described above, the surface emitting laser 1 enables higher uniformity of the density of the current flowing through the inside of the aperture and reduces the electric resistance. In the surface emitting laser 1, since the phase with respect to the resonance wavelength is shifted between the inner side and the outer side of the ridge structure 350, an effective refractive index difference occurs so that light can be confined in the lateral direction. With an enhancement of the confinement of light in the lateral direction, the single mode property in the lateral direction is increased. Further, using a refractive index difference for confining light in the lateral direction allows a reduction in the loss due to diffraction and facilitates a single mode at a low threshold.

Notably, the second layer 320 may not be etched during the formation of the ridge structure 350 as long as the conductive layer 40 is in contact with the interface between the first layer 310 and the second layer 320. In other words, the second layer 320 may not be etched as long as the second layer 320 is exposed by etching the first layer 310. However, when a part or all of the second layer 320 is also etched after etching the first layer 310, the interface between the first layer 310 and the second layer 320 clearly appears on the side wall surface of the ridge structure 350, and the conductive layer 40 is easily brought into contact with this interface. This means that it is more preferable to etch not only the first layer 310 but also the second layer 320.

Further, the material of the first layer 310 is not limited to InGaN, and the first layer 310 may be a p-type GaN layer. Typically, the first layer 310 is an InₐGa₁₋ₐN layer (0 ≤ a < 1), and the second layer 320 is an Al_{b}Ga_{1-b}N layer (0 < b < 1). When the material of the first layer 310 is GaN, crystal defects at the interface between the first layer 310 and the second layer 320 is easily reduced, and effectively high mobilities in the 2DHG is easily obtained. When the material of the first layer 310 is InGaN, a high-concentration 2DHG is easily obtained. The material of the dielectric layer 60 is not limited to SiO₂, and the dielectric layer 60 may be a SiNx layer, a TaOx layer, or the like.

Although the first layer 310 and the second layer 320 are provided in the resonator 20 in the first embodiment, the first layer and the second p-layer may be included in the reflecting mirror by forming a part or all of the upper reflecting mirror as a semiconductor DBR and adjusting the thicknesses of the first layer and the second layer so as to make the first layer and the second layer serve as a part of the semiconductor DBR.

### Second Embodiment

A second embodiment will be described. The second embodiment differs from the first embodiment mainly in the configuration of the ridge structure. FIGS. 3A and 3B are cross-sectional views of an area in and around a ridge structure of a surface emitting laser according to the second embodiment of the present disclosure. FIG. 3B is a cross-sectional view of a portion surrounded by a broken-line circle in FIG. 3A, illustrating currents indicated by arrows in and around the portion.

As illustrated in FIGS. 3A and 3B, in the surface emitting laser according to the second embodiment, the first layer 310 includes a first p-type semiconductor layer 311 and an undoped semiconductor layer 312. The undoped semiconductor layer 312 is on the second layer 320, and the first p-type semiconductor layer 311 is on the undoped semiconductor layer 312. The undoped semiconductor layer 312 is, for example, an In_{0.07}Ga_{0.93}N layer.

The undoped semiconductor layer 312 has a thickness of, for example, 2.5 nm. The first p-type semiconductor layer 311 is a p-type In_{0.07}Ga_{0.93}N layer doped with, for example, Mg at a concentration of 2 × 10¹⁹/cm³. The first p-type semiconductor layer 311 has a thickness of, for example, 12.5 nm.

However, an outermost layer portion at a depth of 10 nm from the upper surface of the first p-type semiconductor layer 311 is doped with Mg at a higher concentration, for example, at a concentration of 3 × 10²⁰/cm³. The band gap of the second layer 320 is larger than the band gap of the first layer 310.

The other configurations are similar to those in the first embodiment.

In the surface emitting laser according to the second embodiment, the undoped semiconductor layer 312 (for example, an In_{0.07}Ga_{0.93}N layer) is below the first p-type semiconductor layer 311 (for example, an In_{0.07}Ga_{0.93}N layer), and the second layer 320 (for example, an Al_{0.05}Ga_{0.95}N layer) is below the undoped semiconductor layer 312. In addition, the band gap of the second layer 320 is larger than that of the first layer 310. This forms a 2DHG in the vicinity of the interface between the first layer 310 and the second layer 320. The 2DHG includes high-concentration carriers, which are two-dimensionally formed in a narrow region having a thickness of approximately 1 nm in the undoped semiconductor layer 312. The conductive layer 40 is in contact with the entire upper surface of the first layer 310 and the interface between the first layer 310 and the second layer 320.

As illustrated in FIG. 3B, current is injected from the conductive layer 40 into the resonator 20 not only from the upper surface of the first layer 310 but also from the interface between the first layer 310 and the second layer 320. In other words, current is injected from the conductive layer 40 to the resonator 20 through the upper surface and the side wall surface of the ridge structure 350. In addition, since the electric resistance of the 2DHG is lower than the electric resistances of the first layer 310 and the second layer 320, the mobility of carriers in the undoped semiconductor layer 312, for which unit is m²/(Vs), is expected to be about two digits. When the concentration of the 2DHG is 1 × 10¹⁹/cm³ or more, a region having a resistance lower than that of the p-GaN layer (the mobility of carriers is typically one rigid when the carrier concentration is on the order of 1 × 10¹⁷) by three or more orders is formed. In this configuration, the current injected from the upper surface of the ridge structure 350 spreads in the lateral direction at the 2DHG portion, and the current injection density tends to become uniform inside the aperture. In other words, the variation in the current injection density inside the ridge structure 350 is reduced.

The second embodiment also attains effects similar to those of the first embodiment.

Notably, the second layer 320 may not be etched during the formation of the ridge structure 350 as long as the conductive layer 40 is in contact with the interface between the undoped semiconductor layer 312 and the second layer 320. In other words, the second layer 320 may not be etched as long as the second layer 320 is exposed by etching the undoped semiconductor layer 312. However, when the second layer 320 is also etched after etching the undoped semiconductor layer 312, the interface between the undoped semiconductor layer 312 and the second layer 320 clearly appears on the side wall surface of the ridge structure 350, and the conductive layer 40 is easily brought into contact with this interface. This means that it is more preferable to etch not only the first layer 310 but also the second layer 320.

Further, the material of the first p-type semiconductor layer 311 is not limited to InGaN, and the first p-type semiconductor layer 311 may be a p-type GaN layer. In this case, the undoped semiconductor layer 312 is preferably an In_{0.08}Ga_{0.92}N layer. The 2DHG is easily formed by reducing the band gap (a third band gap) of the undoped semiconductor layer 312 to be smaller than the band gap (the first band gap) of the first p-type semiconductor layer 311. As described above, it is preferable that the band gap of the second layer 320 is larger than the band gap of the first layer 310, and the band gap of the first p-type semiconductor layer 311 is equal to or larger than the band gap of the undoped semiconductor layer 312. Typically, the first p-type semiconductor layer 311 and the undoped semiconductor layer 312 are InₐGa₁₋ₐN layers (0 ≤ a < 1), and the second layer 320 is an Al_{b}Ga_{1-b}N layer (0 < b < 1).

### Third Embodiment

A third embodiment will be described. The third embodiment differs from the second embodiment mainly in the configuration of the insulating region. FIG. 4 is a cross-sectional view of an area in and around a ridge structure of a surface emitting laser according to a third embodiment of the present disclosure.

As illustrated in FIG. 4, the surface emitting laser according to the third embodiment includes an inactive semiconductor layer 321 instead of the dielectric layer 60. The inactive semiconductor layer 321 contains an impurity element that inactivates an acceptor and is an electrically insulating layer serving as an insulating region that electrically insulates the conductive layer 40 from a portion outside the ridge structure 350 in a plan view of the laminated body of the first layer and the second layer.

The inactive semiconductor layer 321 is formed by, for example, injecting an impurity element that inactivates the acceptor into the surface layer of the second layer 320 while leaving the resist mask used for forming the ridge structure 350, after forming the ridge structure 350. As the impurity element that inactivates the acceptor, an element that serves to inactivate the acceptor by bonding with the p-type dopant or forming a deep level is used. Examples of such element include B, H, and Fe.

Other configurations are the same as those of the second embodiment. As illustrated in FIG. 5, the insulating region (the inactive semiconductor layer 321) has an upper surface closer to the active layer 200 (see FIG. 1) than the interface between the first layer 310 and the second layer 320 in the thickness direction parallel to the direction perpendicular to the upper surface of the first layer 310.

The third embodiment also attains effects similar to those of the second embodiment. Further, the formation of the dielectric layer 60 may be omitted.

### Fourth Embodiment

A fourth embodiment will be described. The fourth embodiment differs from the second embodiment mainly in the configuration of the conductive layer. FIG. 5 is a cross-sectional view of an area in and around a ridge structure of a surface emitting laser according to a fourth embodiment of the present disclosure.

As illustrated in FIG. 5, in the surface emitting laser according to the fourth embodiment, the conductive layer 40 includes a transparent conductive layer 41 and a metal layer 42. A metal layer 42 is on the dielectric layer 60, and the transparent conductive layer 41 is on the metal layer 42. The metal layer 42 is in direct contact with the interface between the first layer 310 and the second layer 320. The metal layer 42 is, for example, an Au layer. The transparent conductive layer 41 is, for example, an ITO layer. For example, the upper surface of the metal layer 42 is substantially flush with the upper surface of the first layer 310. The transparent conductive layer 41 is an example of a first conductive layer, and the metal layer 42 is an example of a second conductive layer.

In manufacturing the surface emitting laser according to the fourth embodiment, for example, after the dielectric layer 60 is formed, the metal layer 42 is formed following the dielectric layer 60 while leaving the resist mask used for forming the ridge structure 350, and then the resist mask is removed. At this time, the thickness of the metal layer 42 is set such that the upper surface of the metal layer 42 is substantially flush with the upper surface of the ridge structure 350. After the lift-off, the transparent conductive layer 41 is formed to be in contact with the upper surface of the ridge structure 350.

Other configurations are the same as those of the second embodiment.

The fourth embodiment also attains effects similar to those of the second embodiment. Further, since the electric resistance of the metal layer 42 is lower than the electric resistance of the transparent conductive layer, the electric resistance of the entire surface emitting laser can be further reduced. Further, since light is blocked by the metal layer 42 covering the outer surface of the ridge structure 350, light is easily confined in the lateral direction.

### Fifth Embodiment

A fifth embodiment will be described. The fifth embodiment differs from the fourth embodiment mainly in the configuration of the conductive layer. FIGS. 6A and 6B are cross-sectional views of an area in and around a ridge structure of a surface emitting laser according to the fifth embodiment of the present disclosure. FIG. 6B is a cross-sectional view of a portion surrounded by a broken-line circle in FIG. 6A, illustrating currents indicated by arrows in and around the portion.

As illustrated in FIG. 6A, in the surface emitting laser according to the fifth embodiment, the conductive layer 40 includes a first transparent conductive layer 43 and a second transparent conductive layer 44. The second transparent conductive layer 44 is on the dielectric layer 60, and the first transparent conductive layer 43 is on the second transparent conductive layer 44. The second transparent conductive layer 44 is in direct contact with the interface between the first layer 310 and the second layer 320. The first transparent conductive layer 43 and the second transparent conductive layer 44 are, for example, ITO layers. The electric resistance of the second transparent conductive layer 44 is lower than the electric resistance of the first transparent conductive layer 43. For example, the upper surface of the second transparent conductive layer 44 is substantially flush with the upper surface of the first layer 310. The first transparent conductive layer 43 is an example of a first conductive layer, and the second transparent conductive layer 44 is an example of a second conductive layer.

In manufacturing the surface emitting laser according to the fifth embodiment, for example, after the dielectric layer 60 is formed, the second transparent conductive layer 44 is formed following the dielectric layer 60 while leaving the resist mask used for forming the ridge structure 350, and then the resist mask is removed. At this time, the thickness of the second transparent conductive layer 44 is set such that the upper surface of the second transparent conductive layer 44 is substantially flush with the upper surface of the ridge structure 350. After the lift-off, the first transparent conductive layer 43 is formed to be in contact with the upper surface of the ridge structure 350.

The other configurations are similar to those in the fourth embodiment.

The fifth embodiment also attains effects similar to those of the fourth embodiment. In addition, the first transparent conductive layer 43 may be made of a material such as ITO that is less likely to absorb light and is likely to have good flatness, whereas the second transparent conductive layer 44 may be made of a material such as ITO that is more likely to absorb light than the first transparent conductive layer 43 or has low electric resistance even if the flatness is low. With this configuration, the electric resistance of the second transparent conductive layer 44 is lower than the electric resistance of the first transparent conductive layer 43, and as illustrated in FIG. 6B, current is easily injected from the conductive layer 40 to the resonator 20 through the interface between the first layer 310 and the second layer 320. This allows a much lower electrical resistance of the surface emitting laser as a whole.

The fifth embodiment enables a reduction in the loss due to diffraction and thus easily provides a single mode at a low threshold more than the fourth embodiment.

### Sixth Embodiment

A sixth embodiment will be described. The sixth embodiment relates to a HUD, which is an example of a projection apparatus. FIG. 7 is a diagram of a HUD as an example of a projection apparatus according to a sixth embodiment. FIG. 8 is a diagram of a vehicle mounted with the HUD according to the sixth embodiment in FIG. 7.

The projection apparatus is a device that projects an image by optical scanning and is, for example, a HUD.

As illustrated in FIG. 8, the HUD 500 according to the sixth embodiment is mounted in the vicinity of a windshield 401 of a vehicle 400, for example. Light L, which has been projected from the HUD 500, is reflected by the windshield 401, and the reflected light is directed to an observer (i.e., a driver 402, or a user). Thus, the driver 402 can visually recognize the image or the like projected by the HUD 500 as a virtual image. Alternatively, a combiner may be disposed on the inner wall surface of the windshield 401 so that the user can visually recognize a virtual image formed by the projection light that is reflected by the combiner. The vehicle 400 is an example of a mobile object.

As illustrated in FIG. 7, the HUD 500 emits three-color laser beams from red, green, and blue laser sources 501R, 501G, and 501B, respectively. The emitted laser beams pass through an incident optical system and then are deflected by a movable device 513 (or an optical deflector) with the reflecting surface 514. The incident optical system includes collimator lenses 502, 503, and 504 respectively provided for the laser sources 501R, 501G, and 501B, two dichroic mirrors 505 and 506, and a light-intensity adjuster 507. The deflected laser beams pass through a projection optical system and are projected onto a screen. The projection optical system (an optical system, of the image projection apparatus) includes a free-form surface mirror 509, an intermediate screen 510, and a projection mirror 511. In the HUD 500, the laser sources 501R, 501G, and 501B, the collimator lenses 502, 503, and 504, and the dichroic mirrors 505 and 506 are unitized as a light source unit 530 in an optical housing. The laser sources 501R, 501G, and 501B include the surface emitting laser according to any one of the first to fifth embodiments. Each of the laser sources 501R, 501G, and 501B may include a surface emitting laser array including multiple surface emitting lasers according to any one of the first to fifth embodiments.

The HUD 500 projects an intermediate image displayed on the intermediate screen 510 onto the windshield 401 of the vehicle 400 to allow the driver 402 to visually recognize the intermediate image as a virtual image.

The laser beams of RGB colors emitted from the laser sources 501R, 501G, and 501B are approximately collimated by the collimator lenses 502, 503, and 504, and are combined by the two dichroic mirrors 505 and 506 each serving as an optical combiner. The light intensity of the combined laser beams is adjusted by the light-intensity adjuster 507, before being deflected for two-dimensionally scanning by the movable device 513 including the reflecting surface 514. The light L being deflected for two-dimensional scanning by the movable device 513 is reflected by the free-form surface mirror 509 and has its distortion corrected, thus being condensed on the intermediate screen 510. The intermediate screen 510 includes a microlens array in which microlenses are two-dimensionally arranged and enlarges the projection light L incident on the intermediate screen 510 in units of microlens.

The movable device 513 causes the reflecting surface 514 to biaxially reciprocate and two-dimensionally scan with the light L incident on the reflecting surface 514. The operation of the movable device 513 is controlled in synchronization with the light-emitting timings of the laser sources 501R, 501G, and 501B.

The operation of the light source unit 530 and the movable device 513 is controlled by the control device 515.

In the above description, the HUD 500 is described as an example of the projection apparatus. However, no limitation is indicated thereby, and the projection apparatus may be any apparatus that performs optical scanning, using the movable device 513 provided with the reflecting surface 514, to project an image.

For example, the present disclosure is also applicable to a projector that is placed on a desk or the like and projects an image on a display screen, a HUD that is incorporated in a wearable member on the head of the observer, for example, and that projects an image on a reflective- and-transmissive screen of the wearable member or on an eyeball as a screen, and the like.

The projection device may be incorporated in, not only the vehicle or the wearable member, but also, for example, a mobile object such as an aircraft, a ship, or a mobile robot; or an immobile object such as an operation robot that operates a driving target such as a manipulator without moving from the installed location.

### Seventh Embodiment

Next, a seventh embodiment will be described. The seventh embodiment relates to an HMD. FIG. 9 is a perspective view of the external appearance of the HMD according to the seventh embodiment.

The HMD is a head-mounted display that can be mounted on a human head, and can be shaped like, for example, glasses.

In FIG. 9, the HMD 600 of the seventh embodiment includes a pair of a front part 600a and a temple 600b respectively provided on the left and right sides. The pairs of the front part 600a and the temple 600b are substantially symmetrical about the center of the HMD 600. The front parts 600a each have a light guide plate 610. An optical system, a controller, and another component are incorporated in the temple 600b.

FIG. 10 is an illustration of a configuration of a part of the HMD 600. Although the configuration for the left eye is illustrated in FIG. 10, the HMD 600 has a configuration similar to that for the right eye.

The HMD 600 includes a control device 515, a light source unit 530, a light-intensity adjuster 507, a movable device 513 including a reflecting surface 514, a light guide plate 610, and a semi-reflective mirror 620.

The light source unit 530 includes, as described above, the laser sources 501R, 501G, and 501B (the surface emitting lasers), the collimator lenses 502, 503, and 504, and the dichroic mirrors 505 and 506, and these elements are combined as a single unit in the optical housing. In the light source unit 530, the laser beams of the RGB colors that are emitted from the laser sources 501R, 501G, and 501B are combined by the two dichroic mirrors 505 and 506. The combined parallel light is emitted from the light source unit 530.

The light intensity of the combined laser beams from the light source unit 530 is adjusted by the light-intensity adjuster 507. Then, the adjusted light is incident on the movable device 513. The movable device 513 moves the reflecting surface 514 in the XY-direction based on the signal from the control device 515 and performs two-dimensional scanning with the light emitted from the light source unit 530. The driving of the movable device 513 is controlled in synchronization with the light emission timings of the laser sources 501R, 501G, and 501B, and a color image is formed with the scanning light.

The scanning light by the movable device 513 is incident on the light guide plate 610. The light guide plate 610 reflects the scanning light on the inner wall and guides the scanning light to the semi-reflective mirror 620. The light guide plate 610 is formed from a material such as a resin having transparency at the wavelength of the scanning light.

The semi-reflective mirror 620 reflects the light guided through the light guide plate 610 to the back side of the HMD 600, emitting the light in the direction to an eye of a wearer 630 of the HMD 600. The semi-reflective mirror 620 has, for example, a free-form surface shape. An image formed of the scanning light is reflected by the semi-reflective mirror 620, thus being formed on the retina of wearer 630. Alternatively, the reflection at the semi-reflective mirror 620 and the effect of the crystalline lenses of eyeballs causes the image of the scanning light to be formed on the retina of the wearer 630. Further, the reflection at the semi-reflective mirror 620 can correct spatial distortion of the image. The wearer 630 can observe an image formed by the light of scanning in the XY direction.

The wearer 630 visually identify an image of external light superposed on the image of the scanning light because the semi-reflective mirror 620 is mounted on each of the front parts 600a. Alternatively, a mirror may be provided instead of the semi-reflective mirror 620 so that external light is blocked out and the wearer 63 observes only the image of the scanning light.

### Eighth Embodiment

The eighth embodiment will be described. The eighth embodiment relates to an optometry apparatus. FIG. 11 is a block diagram of an optometry apparatus according to an eighth embodiment.

The optometric apparatus 800 according to the eighth embodiment represents an apparatus capable of performing various inspections, such as an eyesight inspection, an ocular refractive-power inspection, an ocular tension inspection, and an ocular axial length inspection. The optometric apparatus 800 can perform a non-contact examination on an eye 830 and includes a light source unit 810, a control unit 821, an input unit 822, a storage unit 823, and a display unit 824. The light source unit 810 includes a light source 811 and a projection optical system 812. At the time of optometry, the control unit 821 controls the light source 811, and the projection optical system 812 projects light emitted from the light source 811 onto the eyeball 830 of the subject. In other words, the light source unit 810 emits light containing inspection information onto the eyeball 830 of the subject. The input unit 822 is a device to which a subject or an operator inputs information used for optometry. Information input to the input unit 822 is transmitted to the control unit 821. The storage unit 823 stores the information output by the control unit 821. The display unit 824 displays information output by the control unit 821. The subject fixates his or her face on a supporting section that supports the subject's face and gazes at the test information projected by the projection optical system 812 through the optometric window. The light source 811 used to project the test information from the light source unit 810 includes a surface emitting laser according to any one of the first to fifth embodiments. The light source 811 used to project the test information may include a surface emitting laser array including multiple surface emitting lasers according to any one of the first to fifth embodiments. The optometry apparatus may be an optometry apparatus in the form of glasses. The optometric apparatus in the form of glasses eliminates the needs for space and a large-sized optometric apparatus for examination and enables examination with a simple configuration without being affected by a place.

Although the desirable embodiments and so forth have been described in detail, the present disclosure is not limited to the above-described embodiments and so forth, and various modifications and substitutions can be made without departing from the scope as set forth in the claims.

The above-described embodiments are illustrative and do not limit the present invention. Thus, numerous additional modifications and variations are possible in light of the above teachings. For example, elements and/or features of different illustrative embodiments may be combined with each other and/or substituted for each other within the scope of the present invention.

The scope of the invention is defined by the independent claims.

This patent application is based on and claims priority to Japanese Patent Application No. 2022-044205, filed on March 18, 2022 and Japanese Patent Application No. 2022-192216, filed on November 30, 2022 , in the Japan Patent Office.

### [Reference Signs List]

1 Surface emitting laser
20 Resonator
40 Conductive layer
41 Transparent conductive layer
42 Metal layer
43 First transparent conductive layer
44 Second transparent conductive layer
310 First layer
311 First p-type semiconductor layer
312 Undoped semiconductor layer
320 Second layer
321 Inactive semiconductor layer
330 Third layer

## Claims

1. A surface emitting laser (1) comprising:
a first reflector (10);
a second reflector (30);
a resonator (20) between the first reflector (10) and the second reflector (30), the resonator (20) including an active layer (200); and
a conductive layer (40) through which a current is injected into the active layer (200) of the resonator (20),
wherein the resonator (20) further includes:
a first layer (310) including a first p-type semiconductor layer (311) having a first band gap, the first layer having a first face contacting the conductive layer (40) and a second face opposite to the first face; and
a second layer (320) including a second p-type semiconductor layer having a second band gap, the second layer (320) between the first layer (310) and the active layer (200), the second p-type semiconductor layer contacts the second face of the first layer, and
the conductive layer (40) contacts at least a part of the first face of the first layer (310) and an interface between the first layer (310) and the second layer (320),
**characterized in that**;
the second band gap is larger than the first band gap.

2. The surface emitting laser (1) according to claim 1,
wherein the first layer (310) further includes an undoped semiconductor layer (312) having a third band gap equal to or lower than the first band gap of the first p-type semiconductor layer (311); and
the undoped semiconductor layer (312) has the second face contacting the second layer (320).

3. The surface emitting laser (1) according to claim 1 or 2,
wherein a laminated body, in which the first layer (310) and a portion of the second layer (320) is laminated in a lamination direction, has a ridge structure (350) having a columnar shape,
the first layer (310) has a first outer periphery (700),
the active layer (200) has a second outer periphery (710),
the first outer periphery (700) of the first layer (310) is interior of the second outer periphery (710) of the active layer (200) in a radial direction of the ridge structure (350).

4. The surface emitting laser (1) according to claim 3, further comprising an insulating region between the conductive layer (40) and the second layer (320),
wherein the insulating region is configured to electrically insulate the conductive layer (40) from another portion of the second layer (320) outside the ridge structure (350) in the radial direction.

5. The surface emitting laser (1) according to claim 4,
wherein the ridge structure (350) includes:
the first layer (310); and
the second layer (320), and
the insulating region includes a dielectric layer (60), wherein an upper surface of the dielectric layer (60) is located closer to the active layer (200) than is the interface between the first layer (310) and the second layer (320) in the lamination direction.

6. The surface emitting laser (1) according to claim 4,
wherein the ridge structure (350) includes an entirety of the first layer (310),
the insulating region is closer to the active layer (200) than the interface between the first layer (310) and the second layer (320) in the lamination direction, and
the insulating region includes an inactive semiconductor layer (321) containing an impurity element that inactivates an acceptor.

7. The surface emitting laser (1) according to any one of claims 1 to 6,
wherein the conductive layer (40) includes:
a first conductive layer (41, 43) contacting the first surface of the first layer (310) and having a first electric resistance; and
a second conductive layer (42, 44) contacting the interface between the first layer (310) and the second layer (320) and having a second electric resistance lower than the first electric resistance.

8. The surface emitting laser (1) according to claim 7,
wherein the second conductive layer is a metal layer (42).

9. The surface emitting laser (1) according to any one of claims 1 to 8,
wherein the first p-type semiconductor layer (311) is an InₐGa₁₋ₐN layer (0 ≤ a < 1), and
the second p-type semiconductor layer is an Al_{b}Ga_{1-b}N layer (0 < b < 1).

10. A projection apparatus comprising:
the surface emitting laser (1) according to any one of claims 1 to 9; and
an optical deflector configured to deflect light emitted from the surface emitting laser (1) to project the light onto an object.

11. A head-up display (500) comprising:
the surface emitting laser (1) according to any one of claims 1 to 9.

12. A mobile object (400) comprising:
the head-up display (500) according to claim 11.

13. A head-mounted display (600) comprising:
the surface emitting laser (1) according to any one of claims 1 to 9.

14. An optometry apparatus (800) comprising:
the surface emitting laser (1) according to any one of claims 1 to 9.

## Patentansprüche

1. Oberflächenemittierender Laser (1), umfassend:
einen ersten Reflektor (10);
einen zweiten Reflektor (30);
einen Resonator (20) zwischen dem ersten Reflektor (10) und dem zweiten Reflektor (30), wobei der Resonator (20) eine aktive Schicht (200) beinhaltet; und
eine leitende Schicht (40), durch die ein Strom in die aktive Schicht (200) des Resonators (20) injiziert wird,
wobei der Resonator (20) ferner beinhaltet:
eine erste Schicht (310), die eine erste Halbleiterschicht (311) des p-Typs beinhaltet, die eine erste Bandlücke aufweist, wobei die erste Schicht eine erste Fläche, die die leitende Schicht (40) berührt, und eine zweite Fläche aufweist, die der ersten Fläche gegenüberliegt; und
eine zweite Schicht (320), die eine zweite Halbleiterschicht des p-Typs beinhaltet, die eine zweite Bandlücke aufweist, wobei die zweite Schicht (320) zwischen der ersten Schicht (310) und der aktiven Schicht (200) liegt, die zweite Halbleiterschicht des p-Typs die zweite Fläche der ersten Schicht berührt, und
die leitende Schicht (40) wenigstens einen Teil der ersten Fläche der ersten Schicht (310) und eine Grenzfläche zwischen der ersten Schicht (310) und der zweiten Schicht (320) berührt,
**dadurch gekennzeichnet, dass**;
die zweite Bandlücke größer ist als die erste Bandlücke.

2. Oberflächenemittierender Laser (1) nach Anspruch 1,
wobei die erste Schicht (310) ferner eine undotierte Halbleiterschicht (312) mit einer dritten Bandlücke beinhaltet, die gleich oder kleiner als die erste Bandlücke der ersten Halbleiterschicht (311) des p-Typs ist; und
die undotierte Halbleiterschicht (312) die zweite Fläche aufweist, die die zweite Schicht (320) berührt.

3. Oberflächenemittierender Laser (1) nach Anspruch 1 oder 2,
wobei ein laminierter Körper, in dem die erste Schicht (310) und ein Abschnitt der zweiten Schicht (320) in einer Laminierrichtung laminiert sind, eine Rippenstruktur (350) mit einer säulenartigen Form aufweist,
die erste Schicht (310) einen zweiten Außenumfang (700) aufweist,
die aktive Schicht (200) einen zweiten Außenumfang (710) aufweist,
der erste Außenumfang (700) der ersten Schicht (310) innerhalb des zweiten Außenumfangs (710) der aktiven Schicht (200) in einer radialen Richtung der Rippenstruktur (350) liegt.

4. Oberflächenemittierender Laser (1) nach Anspruch 3, der ferner einen isolierenden Bereich zwischen der leitenden Schicht (40) und der zweiten Schicht (320) umfasst,
wobei der isolierende Bereich dazu konfiguriert ist, die leitende Schicht (40) elektrisch von einem anderen Abschnitt der zweiten Schicht (320) außerhalb der Rippenstruktur (350) in der radialen Richtung zu isolieren.

5. Oberflächenemittierender Laser (1) nach Anspruch 4,
wobei die Rippenstruktur (350) umfasst:
die erste Schicht (310); und
die zweite Schicht (320), und
der isolierende Bereich eine dielektrische Schicht (60) beinhaltet, wobei sich eine obere Oberfläche der dielektrischen Schicht (60) näher an der aktiven Schicht (200) befindet als die Grenzfläche zwischen der ersten Schicht (310) und der zweiten Schicht (320) in der Laminierungsrichtung.

6. Oberflächenemittierender Laser (1) nach Anspruch 4,
wobei die Rippenstruktur (350) eine Gesamtheit der ersten Schicht (310) beinhaltet,
der isolierende Bereich näher an der aktiven Schicht (200) liegt als die Grenzfläche zwischen der ersten Schicht (310) und der zweiten Schicht (320) in der Laminierungsrichtung, und
der isolierende Bereich eine inaktive Halbleiterschicht (321) beinhaltet, die ein Verunreinigungselement enthält, das einen Akzeptor inaktiviert.

7. Oberflächenemittierender Laser (1) nach einem der Ansprüche 1 bis 6,
wobei die leitfähige Schicht (40) umfasst:
eine erste leitende Schicht (41, 43), die die erste Oberfläche der ersten Schicht (310) berührt und einen ersten elektrischen Widerstand aufweist; und
eine zweite leitfähige Schicht (42, 44), die die Grenzfläche zwischen der ersten Schicht (310) und der zweiten Schicht (320) berührt und einen ersten elektrischen Widerstand aufweist, der niedriger als der erste elektrische Widerstand ist.

8. Oberflächenemittierender Laser (1) nach Anspruch 7,
wobei die zweite leitende Schicht eine Metallschicht (42) ist.

9. Oberflächenemittierender Laser (1) nach einem der Ansprüche 1 bis 8,
wobei die erste Halbleiterschicht (311) des p-Typs eine InₐGa₁₋ₐN-Schicht (0 ≤ a< 1) ist, und die zweite Halbleiterschicht des p-Typs eine Al_{b}Ga_{1-b}N-Schicht ist (0 < b < 1).

10. Projektionseinrichtung, umfassend:
den oberflächenemittierenden Laser (1) nach einem der Ansprüche 1 bis 9; und
eine optische Ablenkeinheit, die dazu konfiguriert ist, das von dem oberflächenemittierenden Laser (1) emittierte Licht abzulenken, um das Licht auf ein Objekt zu projizieren.

11. Head-up-Display (500), umfassend:
den oberflächenemittierenden Laser (1) nach einem der Ansprüche 1 bis 9.

12. Mobiles Objekt (400), umfassend:
den Head-up-Display (500) nach Anspruch 11.

13. Datenhelm (600), umfassend:
den oberflächenemittierenden Laser (1) nach einem der Ansprüche 1 bis 9.

14. Optometrieeinrichtung (800), umfassend:
den oberflächenemittierenden Laser (1) nach einem der Ansprüche 1 bis 9.

## Revendications

1. Laser à émission par la surface (1) comprenant :
un premier réflecteur (10) ;
un deuxième réflecteur (30) ;
un résonateur (20) entre le premier réflecteur (10) et le deuxième réflecteur (30), le résonateur (20) incluant une couche active (200) ; et
une couche conductrice (40) à travers laquelle un courant est injecté dans la couche active (200) du résonateur (20),
dans lequel le résonateur (20) inclut en outre :
une première couche (310) incluant une première couche semi-conductrice de type p (311) présentant une première bande interdite, la première couche présentant une première face en contact avec la couche conductrice (40) et une deuxième face opposée à la première face ; et
une deuxième couche (320) incluant une deuxième couche semi-conductrice de type p présentant une deuxième bande interdite, la deuxième couche (320) se trouvant entre la première couche (310) et la couche active (200), la deuxième couche semi-conductrice de type p étant en contact avec la deuxième face de la première couche, et
la couche conductrice (40) étant en contact avec au moins une partie de la première face de la première couche (310) et une interface entre la première couche (310) et la deuxième couche (320),
**caractérisé en ce que** ;
la deuxième bande interdite est plus grande que la première bande interdite.

2. Laser à émission par la surface (1) selon la revendication 1,
dans lequel la première couche (310) inclut en outre une couche semi-conductrice non dopée (312) présentant une troisième bande interdite inférieure ou égale à la première bande interdite de la première couche semi-conductrice de type p (311) ; et
la couche semi-conductrice non dopée (312) présente la deuxième face en contact avec la deuxième couche (320).

3. Laser à émission par la surface (1) selon la revendication 1 ou 2,
dans lequel un corps stratifié, dans lequel la première couche (310) et une portion de la deuxième couche (320) sont stratifiées dans une direction de stratification, présente une structure en crête (350) présentant une forme colonnaire,
la première couche (310) présente une première périphérie extérieure (700),
la couche active (200) présente une deuxième périphérie extérieure (710),
la première périphérie extérieure (700) de la première couche (310) est à l'intérieur de la deuxième périphérie extérieure (710) de la couche active (200) dans une direction radiale de la structure en crête (350).

4. Laser à émission par la surface (1) selon la revendication 3, comprenant en outre une région isolante entre la couche conductrice (40) et la deuxième couche (320),
dans lequel la région isolante est configurée pour isoler électriquement la couche conductrice (40) d'une autre portion de la deuxième couche (320) à l'extérieur de la structure en crête (350) dans la direction radiale.

5. Laser à émission par la surface (1) selon la revendication 4,
dans lequel la structure en crête (350) inclut :
la première couche (310) ; et
la deuxième couche (320), et
la région isolante inclut une couche diélectrique (60), dans lequel une surface supérieure de la couche diélectrique (60) est située plus près de la couche active (200) que ne l'est l'interface entre la première couche (310) et la deuxième couche (320) dans la direction de stratification.

6. Laser à émission par la surface (1) selon la revendication 4,
dans lequel la structure en crête (350) inclut la totalité de la première couche (310),
la région isolante est située plus près de la couche active (200) que l'interface entre la première couche (310) et la deuxième couche (320) dans la direction de stratification, et
la région isolante inclut une couche semi-conductrice inactive (321) contenant un élément d'impureté qui désactive un accepteur.

7. Laser à émission par la surface (1) selon l'une quelconque des revendications 1 à 6,
dans lequel la couche conductrice (40) inclut :
une première couche conductrice (41, 43) en contact avec la première surface de la première couche (310) et présentant une première résistance électrique ; et
une deuxième couche conductrice (42, 44) en contact avec l'interface entre la première couche (310) et la deuxième couche (320) et présentant une deuxième résistance électrique inférieure à la première résistance électrique.

8. Laser à émission par la surface (1) selon la revendication 7,
dans lequel la deuxième couche conductrice est une couche en métal (42).

9. Laser à émission par la surface (1) selon l'une quelconque des revendications 1 à 8,
dans lequel la première couche semi-conductrice de type p (311) est une couche de InₐGa₁₋ₐN (0 ≤ a < 1), et
la deuxième couche semi-conductrice de type p est une couche de Al_{b}Ga_{1-b}N (0 < b < 1).

10. Appareil de projection comprenant :
le laser à émission par la surface (1) selon l'une quelconque des revendications 1 à 9 ; et
un déflecteur optique configuré pour dévier la lumière émise par le laser à émission par la surface (1) afin de projeter la lumière sur un objet.

11. Affichage tête haute (500) comprenant :
le laser à émission par la surface (1) selon l'une quelconque des revendications 1 à 9.

12. Objet mobile (400) comprenant :
l'affichage tête haute (500) selon la revendication 11.

13. Affichage tête haute (600) comprenant :
le laser à émission par la surface (1) selon l'une quelconque des revendications 1 à 9.

14. Appareil d'optométrie (800) comprenant :
le laser à émission par la surface (1) selon l'une quelconque des revendications 1 à 9.
